# EUROPÄISCHE PATENTSCHRIFT

(11) **EP 2 570 800 B1**
(45) Veröffentlichungstag und Bekanntmachung des Hinweises auf die Patenterteilung: **27.01.2021**
(21) Anmeldenummer: 11181718.5
(22) Anmeldetag: 16.09.2011
(51) Int. Cl.: G01N 21/84, G01N 33/487, G01N 35/00

(54) **Testbandkassette mit analytischem Testband**
Test strip cassette with analytical test strip
Cassette à bande de test avec bande de test analytique

(43) Veröffentlichungstag der Anmeldung: 20.03.2013
(73) Patentinhaber: Roche Diabetes Care GmbH, 68305 Mannheim (DE); F. Hoffmann-La Roche AG, 4070 Basel (CH)
(72) Erfinder: Dreibholz, Joerg, 67122 Altrip (DE); Freitag, Christian, 55278 Winolsheim (DE); Jaeck, Thomas, 68542 Heddesheim (DE); Pachl, Rudolf, 67158 Ellerstadt (DE); Schmidtchen, Elke, 68199 Mannheim (DE); Keth, Ingrid, 67551 Worms (DE); Schwoebel, Wolfgang, 68309 Mannheim (DE); Seelig, Peter, 60596 Frankfurt a.M. (DE)
(74) Vertreter: Pfiz, Thomas

(56) Entgegenhaltungen:
- EP-A1- 1 424 040
- EP-A1- 1 878 379
- EP-A1- 2 116 180
- EP-A1- 2 151 686
- EP-A1- 2 223 746
- EP-A2- 1 702 565
- WO-A1-2005/032372

## Beschreibung

Die Erfindung betrifft eine Testbandkassette zum Einsetzen in ein Handgerät, insbesondere für Blutzuckertests, mit einem Kassettengehäuse zur Lagerung von Bandspulen, einem mittels der Bandspulen vorspulbaren analytischen Testband, einer Vielzahl von auf dem Testband bevorrateten analytischen Testelementen, welche ein Spreitgewebe zur Applikation von Körperflüssigkeit und eine darunter liegende Reagenzschicht zum Nachweis eines Analyten in der Körperflüssigkeit aufweisen, wobei das Kassettengehäuse eine das Testband umlenkende Applikationsspitze zur Bereitstellung der Testelemente besitzt. Solche Kassetten sind aus EP2116180 A1, EP1424040 A1 und EP2223746 A1 bekannt.

Die Erfindung betrifft weiter ein analytisches Testband insbesondere zum Einsatz in einer solchen Testbandkassette.

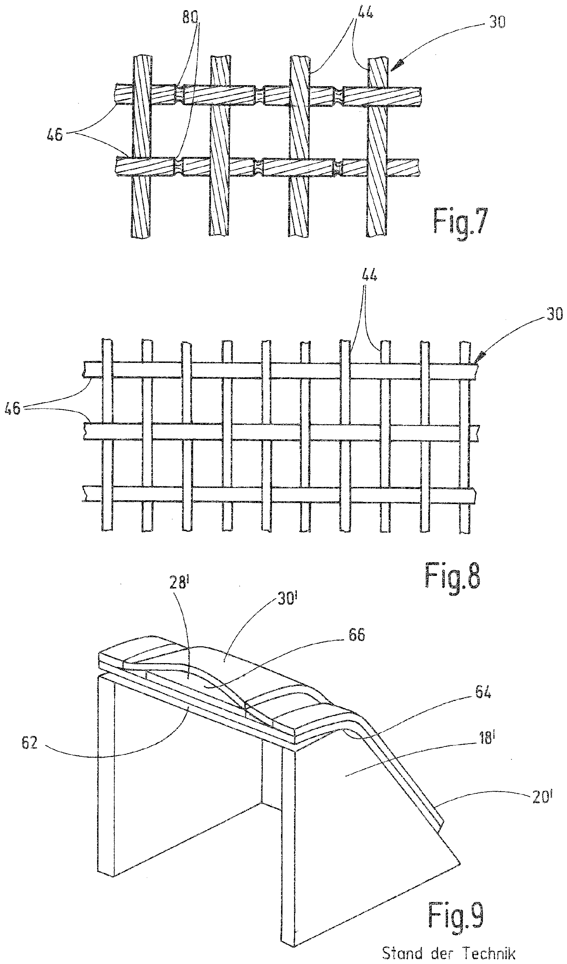

Bei einer aus der EP-A 1 878 379 bekannten Testbandkassette dieser Art weist die Bandführung an einem Messkopf einen ebenen Auflagerahmen auf, welcher ein Testelement an der Messstelle plan aufspannt. Das Testband wird dabei ausgehend von Umlenkschrägen über die in Bandquerrichtung verlaufenden Rahmenschenkel abgeknickt, um eine frei gespannte Planlage zu erreichen. Die zur Messung eingesetzten etikettenartigen Testelemente bzw. Testfelder weisen einen zentralen Chemieträger auf, der an seinen Seitenrändern von dem Spreitgewebe hintergriffen wird. Als möglicher Nachteil hat sich dabei herausgestellt, dass unter den herrschenden Belastungen des Testbandaufbaus auf dem ebenen Auflagerahmen eine Spaltbildung zwischen dem Spreitnetz und dem Chemieträger auftreten kann, wie es in der Zeichnung in Fig. 9 veranschaulicht ist. Das Spaltmaß ist dabei im Zentrum maximal und fällt zu den Seiten hin ab, was unterschiedliche Kapillarkräfte erzeugen kann und letztlich unerwünschte Verteilungen (ggf. mit Vorzugsrichtung) des Messmediums zur Folge haben kann.

Insbesondere wurde festgestellt, dass eine solche Spaltbildung die Neigung zu einer "Entnetzung", d.h. einer Migration der Blutprobe aus den benetzten Gewebemaschen und die Empfindlichkeit gegenüber Verunreinigungen (z.B. Glucose) auf der Haut des Probanden verstärkt. Der letztgenannte Effekt kann sich dadurch ergeben, dass das durch einen Hauteinstich gewonnene Kapillarblut einen Blutstropfen an der Einstichstelle bildet, der zunächst in seinem im Hautkontakt stehenden Randbereich mit Verschmutzungen aufkonzentriert wird. Das "native" Blut wird dann aufgrund der Spaltbildung im Spreitgewebe zunächst in die Randbereiche des Chemieträgers verteilt, während das nachströmende verschmutzte Blut dann auf den zentralen Messfleck gelangt, wodurch die Messperformance beeinträchtigt werden kann.

Ausgehend hiervon liegt der Erfindung die Aufgabe zugrunde, die im Stand der Technik bekannten Testbandsysteme und darin eingesetzten disposiblen Testmittel zu optimieren und eine weiter verbesserte Zuverlässigkeit und Genauigkeit der Messung sicherzustellen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Bandführungsgeometrie bzw. den Testfeldaufbau so zu gestalten, dass eine Spaltbildung zwischen Spreitgewebe und Chemieträger unter Einsatzbedingungen weitgehend minimiert wird. Dementsprechend wird gemäß einem ersten Aspekt der Erfindung vorgeschlagen, dass die Applikationsspitze eine längs des Testbandes (bzw. in Bandlaufrichtung) bogenförmig verlaufende und quer dazu ungekrümmte Führungsbahn zur knickfreien Abstützung des Testbands aufweist, und dass die Führungsbahn in ihrem Scheitelbereich einen zentralen Durchbruch als Messfenster für eine optische Messung an den Testelementen begrenzt. Durch die bogenförmige Längskrümmung der Führungsbahn bzw. Lauffläche kann der Biegesteifigkeit der Testelemente und insbesondere des Spreitgewebes durch einen gleichmäßig abstützenden mechanischen Unterbau Rechnung getragen werden, so dass scharfe Bandknicke und damit eine Spaltbildung vermieden werden. Um Verschiebungen in dem Mehrschichtaufbau zu vermeiden, erfolgt die Bandkrümmung nur eindimensional, während in Bandquerrichtung eine ungekrümmte Abstützung erreicht wird. Zugleich kann durch die Bogenform der Führungsbahn eine gezielte Probenaufnahme auch kleinster Probenmengen im Scheitelpunkt sichergestellt werden, wobei durch das dort positionierte, als lichte Öffnung ausgebildete Messfenster auch eine Reduzierung der erforderlichen Testfeldfläche möglich ist.

Eine weitere Verbesserung in dieser Hinsicht lässt sich dadurch erreichen, dass die kreisbogenförmige Führungsbahn einen festen Krümmungsradius vorzugsweise im Bereich von 3 bis 5 mm aufweist, und dass die Führungsbahn eine Längserstreckung in Bandtransportrichtung im Bereich von 5 bis 8 mm aufweist. Dabei ist es günstig, wenn die Längserstreckung der Führungsbahn gleich oder kleiner als die vorzugsweise im Bereich von 5 bis 15 mm liegende Länge der Testelemente ist.

Die Einsatzbedingungen und insbesondere die Bandzugkraft sollten so angepasst sein, dass das Testband unter Zugspannung flächig auf der Führungsbahn abgestützt ist, so dass das Spreitgewebe im Wesentlichen spaltfrei auf der Reagenzschicht anliegt.

Um eine Spaltbildung unter dem unverklebten Zentralbereich des Spreitgewebes zu vermeiden, ist es vorteilhaft, wenn die Reagenzschicht in Bandquerrichtung gesehen schmaler als das Testband und breiter als das Messfenster ist.

Für eine optimierte rückseitige optische Messwerterfassung ist es von Vorteil, wenn eine die Führungsbahn bildende Gehäusewand der Applikationsspitze rückseitig zu dem Messfenster hin abgeschrägt ist.

Um Gebrauchs- und Herstellungsvorteile zu erreichen, ist es vorteilhaft, wenn die Applikationsspitze vorzugsweise als Spritzgusspartie einstückig an dem Kassettengehäuse angeformt ist und im Gebrauchszustand zum punktuellen Applizieren von Körperflüssigkeit aus dem Handgerät herausragt.

Im Folgenden wird die Erfindung anhand der in der Zeichnung schematisch dargestellten Ausführungsbeispiele näher erläutert. Es zeigen
- Fig. 1: ein Handgerät für Blutzuckertests mit einer als Verbrauchsmittel eingesetzten Testbandkassette in einer teilweise aufgebrochenen Seitenansicht;
- Fig. 2: einen Ausschnitt eines Testbands der Testbandkassette mit einem analytischen Testelement in einer perspektivischen Darstellung;
- Fig. 3: ein Gehäuseteil der Testbandkassette in einer perspektivischen Darstellung;
- Fig. 4: eine Applikationsspitze der Testbandkassette in der Draufsicht, im Längsschnitt und im Querschnitt;
- Fig. 5 und 6: einen Schnitt entlang der Linie 5 - 5 und 6 - 6 in Fig. 4;
- Fig. 7: ein Spreitgewebe für ein analytisches Testelement mit Schwächungsstellen in einer ausschnittsweisen Draufsicht;
- Fig. 8: eine weitere Ausführungsform eines modifizierten Spreitgewebes;
- Fig. 9: ein Testfeld auf einer Applikationsspitze gemäß dem Stand der Technik in geschnittener perspektivischer Darstellung.

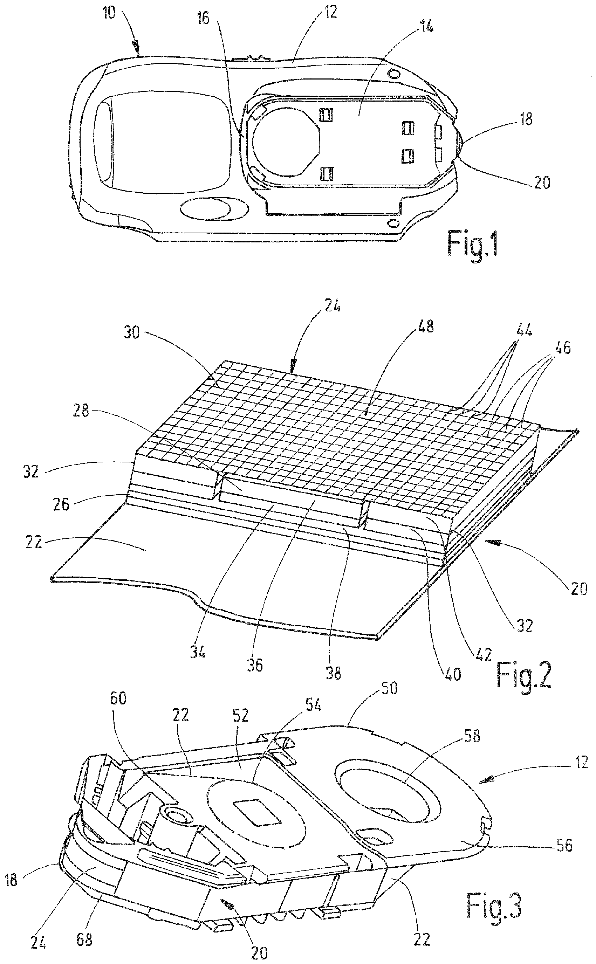

Das in Fig. 1 gezeigte Blutzuckermesssystem 10 ermöglicht speziell für insulinpflichtige Patienten die Durchführung von Glukosebestimmungen an vor Ort durch einen Hauteinstich gewonnenen Blutproben. Zu diesem Zweck ist das Handgerät 12 gleichsam als mobiles Labor in der Hand eines Probanden tragbar und benutzbar. Um die Handhabung weitgehend zu vereinfachen, lässt sich eine Testbandkassette 14 als analytisches Verbrauchsmittel zur Bevorratung einer Vielzahl von Einzeltests in einen Kassettenschacht 16 des Geräts 12 einsetzen, wobei im Gebrauchszustand (bei entfernter Schutzkappe) eine Applikationsspitze 18 frei aus dem Gerät 12 herausragt, so dass dort ein Testband 20 zum oberseitigen Auftrag eines Blutstropfens und zur rückseitigen photometrischen Untersuchung umgelenkt werden kann. Denkbar ist auch die Untersuchung anderer Körperflüssigkeiten, beispielsweise Gewebeflüssigkeit.

Fig. 2 zeigt einen Abschnitt des in der Testbandkassette 14 geführten Testbandes 20. Dieses umfasst ein aufwickelbares flexibles Transportband 22 und eine Vielzahl von darauf für einen sukzessiven Einmalgebrauch bevorrateten, in Bandlängsrichtung voneinander beabstandeten Testelementen 24. Beispielsweise besteht das Transportband 22 aus einer 5 mm breiten und 12 µm dicken Folie, auf die Testelemente 24 mit einer jeweiligen Gesamthöhe von etwa 200 µm aufgebracht sind.

Die mehrlagigen Testelemente 24 weisen als im Umriss rechteckförmige etikettenartige Flächengebilde einen auf das Transportband 22 aufgeklebten doppelseitigen Klebestreifen 26, einen darauf aufgebrachten Chemieträger 28 und ein den Chemieträger 28 an der von dem Transportband 22 abgewandten Oberseite überspannendes Spreitgewebe 30 für eine flächige Verteilung einer von oben auf das Spreitgewebe aufgetragenen Blutprobe auf. Seitlich neben dem Chemieträger 28 sind Abstandshalter 32 vorgesehen, um das Spreitgewebe 30 eben bzw. stufenfrei vollflächig abzustützen.

Der Chemieträger 28 umfasst eine lichtdurchlässige Trägerfolie 34 und eine darauf aufgebrachte Reagenzschicht 36, die aus einer oberen Pigmentschicht mit darunter liegendem Trockenchemiefilm in an sich bekannter Weise aufgebaut ist.

Die Abstandshalter 32 bestehen aus einem auf der oberen Klebeschicht 38 des doppelseitigen Klebestreifens 26 haftenden Basisstreifen 40 und einer darauf befindlichen Klebeschicht 42 zur seitlichen Fixierung des Spreitgewebes 30.

Das in Fig. 2 nur schematisch und in der Dicke nicht maßstäblich gezeigte Spreitgewebe 30 ist durch gitterförmig miteinander verkreuzte Gewebefäden 44, 46 gebildet. Diese können als Kettfäden 44 und Schussfäden 46 in Leinwandbindung miteinander verbunden sein und zur lokalen Modifikation der Biegesteifigkeit uneinheitlich ausgebildet sein, wie es weiter unten näher erläutert wird. Das Spreitgewebe 30, das eine Dicke von etwa 100 µm aufweist, sorgt aufgrund seiner kapillaren Zwischenräume für eine rasche Aufnahme der Flüssigprobe auf der freien Oberseite und für eine flächige Verteilung auf die darunter liegende Reagenzschicht 36.

Dabei wird durch den einseitigen Verschluss der Gewebeöffnungen mit dem Klebematerial der flankierenden Klebeschichten 42 eine Art von Wabenstruktur über den Abstandshaltern 32 gebildet, die einen Blutfluss zu den Seitenkanten des Testelements 24 verhindert. Somit erfolgt die Flüssigkeitsverteilung bzw. -spreitung gezielt in dem nicht verklebten Zentralbereich des Gewebes 30 über der Reagenzschicht 36, so dass auf die nach dem Stand der Technik beispielsweise als Wachsstreifen mittels Thermotransferdruck eigens aufgebrachten hydrophoben Randstreifen ohne Nachteil verzichtet werden kann.

Die Reagenzschicht 36 spricht mit ihrem Trockenchemiefilm insbesondere auf Enzymbasis auf einen Analyten (Glucose) durch Farbumschlag an, so dass eine reflexionsphotometrische Erfassung durch den transparenten Folienverbund 22, 26, 34 hindurch von der Rückseite des Testbands 20 her erfolgen kann.

Fig. 3 zeigt das Kassettengehäuse 50 der Testbandkassette 14 bei abgenommenem Gehäusedeckel. Das Gehäuse 50 umschließt eine Vorratskammer 50 zur abgedichteten Lagerung einer Vorratsspule 54 für unverbrauchtes Testband. An einem Gehäuseflansch 56 ist eine rotierend angetriebene Aufwickelspule 58 zum Aufwickeln von verbrauchtem Testband gelagert. Das Testband 20 wird somit über eine einheitlich durch das Kassettengehäuse 50 gebildete Bandführung von der Vorratsspule 54 abgezogen, über die Applikationsspitze 18 umgelenkt und auf der Aufwickelspule 58 entsorgt, wobei eine Durchzugdichtung 60 an der Vorratskammer 50 für die Aufrechterhaltung einer Bandspannung sorgt.

Die Testfelder 24 lassen sich somit durch Vorspulen des Transportbandes 22 an Applikationsspitze 18 nacheinander zum Einsatz bringen, um gezielt eine geringe Probenmenge auftragen zu können. Aufgrund der dabei ausgeübten Zugkraft ist der mehrlagige Bandaufbau richtungsabhängigen Dehnungen bzw. Kontraktionen insbesondere im Bereich von engen Umlenkstellen unterworfen.

Beim Stand der Technik, wie er in Fig. 9 für eine bekannte Applikationsspitze 18' mit ebenem Auflagerahmen 62 dargestellt ist, kann die Biegesteifigkeit des Spreitgewebes 30' in Bandlängs- und -querrichtung zu einem Abheben bzw. Aufwölben über dem Chemieträger 28' führen. Dieser Effekt beruht einerseits auf Biegungen an den engen Umlenkstellen 64 und auf der Bandwölbung in Querrichtung aufgrund der seitlichen Gewebeverklebung unterhalb der Höhe des Chemieträgers 28'. Dies führt zur Bildung eines Spalts 66 im Zentralbereich zwischen Spreitgewebe 30' und Chemieträger 28', wodurch der Blutübertrag und damit der der Messerfolg beeinträchtigt werden kann.

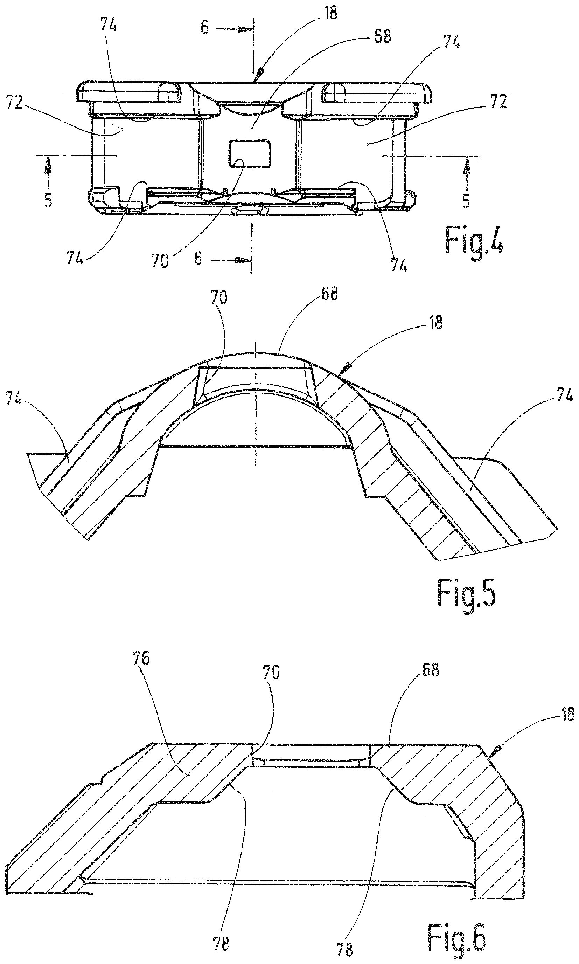

Um die vorstehend beschriebene Spaltbildung zu vermeiden, ist gemäß Fig. 4 bis 6 die Applikationsspitze 18 mit einer in Bandlängsrichtung bzw. Bandtransportrichtung bogenförmig verlaufenden, außen konvexen Führungsbahn 68 für das Testband 20 versehen. In ihrem Scheitelbereich begrenzt die Führungsbahn 68 einen zentralen Durchbruch 70 allseitig als Messfenster für eine rückseitige optische Vermessung der Testelemente 24.

Zur Ein- und Ausleitung des Testbands 20 an der Applikationsspitze 18 schließen sich an die Führungsbahn 68 endseitig Führungsschrägen 72 an, die einen spitzen Winkel einschließen. In diesem Bereich sind auch Seitenbegrenzungen 74 vorgesehen, die das Testband 20 gegen Querverschiebung sichern, während die Führungsbahn 68 frei von solchen Seitenbegrenzungen bleibt.

Wie am besten aus Fig. 5 ersichtlich, weist die kreisbogenförmige Führungsbahn 68 einen vorgegebenen, definierten Krümmungsradius auf, der zweckmäßig im Bereich von 3 bis 5 mm liegt. Entsprechend kann die Führungsbahn 68 eine Längserstreckung in Bandtransportrichtung im Bereich von 5 bis 8 mm besitzen. Dabei ist es zweckmäßig, wenn die Testelemente 24 an die Längserstreckung der Führungsbahn 68 angepasst sind. Besonders vorteilhaft sind die Testelemente 24 zur Vermeidung von Zug- und Schubspannungen durch Umlenkpunkte auf eine möglichst geringe Länge verkürzt.

Wie in Fig. 6 deutlich erkennbar, verläuft die Führungsbahn 68 in Bandquerrichtung gesehen ungekrümmt bzw. linear, so dass das Spreitgewebe 30 nur eindimensional in Bandlängsrichtung gebogen wird und dabei im Wesentlichen spaltfrei auf der Reagenzschicht 36 aufliegt. Zweckmäßig ist die Reagenzschicht 36 breiter als das Messfenster 70, so dass der Messfleck in jedem Fall auf der Reagenzschicht 36 liegt. Für den Strahlengang der photometrischen Messoptik ist die vorderseitig als Führungsbahn 68 ausgebildete Gehäusewand 76 der Applikationsspitze 68 an ihrer Rückseite 78 zu dem Messfenster 70 hin abgeschrägt.

Als weitere Maßnahme zur Vermeidung bzw. Reduzierung einer Spaltbildung zwischen dem Spreitgewebe 30 und der Reagenzschicht 36 ist die Biegesteifigkeit des Spreitgewebes 30 bereichsweise bzw. lokal entsprechend modifiziert.

Allgemein lässt sich die Biegesteifigkeit einer Gewebeprobe nach dem Freiträgerverfahren (Cantilever-Verfahren) bestimmen. Hierbei wird das Biegeverhalten aufgrund des Eigengewichts dadurch ermittelt, dass eine Biegelänge erfasst wird, bei der die Gewebeprobe unter ihrem Eigengewicht um einen definierten Winkel nach unten gebogen wird.

Beispielsweise kann eine hohe Biegesteifigkeit in Bandquerrichtung vorgesehen sein, um eine benötigte Planlage des Spreitgewebes sicherzustellen. Es kann aber auch eine niedrige Biegesteifigkeit in Bandlängsrichtung notwendig sein, um das Spreitgewebe umlenken zu können, ohne dass es zu ungewollten Aufwölbungen oder einer Delaminierung weiterer Teile des gesamten Testelementaufbaus kommt.

Ein isotropes Gewebe mit einheitlichen Schuss- und Kettfäden kann je nach Fadenstärke bzw. Gewebedicke nur eine in Bezug auf die gewünschte Testelementarchitektur gleichmäßig hohe oder niedrige Biegesteifigkeit besitzen.

Um ein für die Testfunktion ausreichend dickes Spreitgewebe mit Bereichen hoher Biegesteifigkeit und zugleich mit Bereichen geringer Biegesteifigkeit zu erhalten, bestehen folgende Möglichkeiten, die Biegesteifigkeit bereichsweise bzw. lokal zu modifizieren:
- der Einsatz unterschiedlich starker Fäden in entsprechenden Bereichen des Gewebes;
- das Herauslösen einzelner Fäden in bestimmten Bereichen;
- die zusätzliche Beschichtung in bestimmten Bereichen;
- die Verdünnung oder lokale Schwächung von einem oder mehreren Fäden;
- der Einsatz unterschiedlicher Fadenmaterialien;
- der Einsatz unterschiedlicher Fadenbeschaffenheiten (z.B. multifile Fäden).

Fig. 7 zeigt ein Beispiel eines Spreitgewebes 30, bei dem nur eines der Fadensysteme 44, 46 (beispielsweise die Schussfäden 46) stellenweise reduzierte Fadenquerschnitte aufweist. Dies kann dadurch erzeugt werden, dass die Gewebefäden 46 in dem vorgefertigten Gewebe 30 durch Laserablation mit Schwächungsstellen 80 versehen werden. Denkbar sind auch Ätzverfahren oder ein mechanischer Materialabtrag beispielsweise mittels einer Wafersäge, um lokale Schwächungsstellen zur Reduzierung der Biegesteifigkeit einzubringen.

Fig. 8 veranschaulicht ein weiteres Beispiel, bei dem das Spreitgewebe 30 durch unterschiedliche Fadenstärken im Bereich seiner beiden Fadensysteme in der Biegesteifigkeit modifiziert ist. Dabei weisen die Kettfäden 44 eine geringere Fadenstärke und demzufolge eine geringere Biegesteifigkeit als die dickeren Schussfäden 46 auf. Möglich ist es auch, dass die Gewebegeometrie des Spreitgewebes 30 beispielsweise durch Herauslösen einzelner Gewebefäden lokal variiert wird.

## Patentansprüche

1. Testbandkassette zum Einsetzen in ein Handgerät (12), insbesondere für Blutzuckertests, mit einem Kassettengehäuse (50) zur Lagerung von Bandspulen (54,58), einem mittels der Bandspulen (54,58) vorspulbaren analytischen Testband (20), einer Vielzahl von auf dem Testband (20) bevorrateten analytischen Testelementen (24), welche ein Spreitgewebe (30) zur Applikation von Körperflüssigkeit und eine darunter liegende Reagenzschicht (36) zum Nachweis eines Analyten in der Körperflüssigkeit aufweisen, wobei das Kassettengehäuse (50) eine das Testband (20) umlenkende Applikationsspitze (18) zur Bereitstellung der Testelemente (24) besitzt, **dadurch gekennzeichnet, dass** die Applikationsspitze (18) eine längs des Testbands (20) bogenförmig verlaufende und quer dazu ungekrümmte Führungsbahn (68) zur knickfreien Abstützung des Testbands (20) aufweist, dass die Führungsbahn (68) in ihrem Scheitelbereich einen zentralen Durchbruch als Messfenster (70) für eine optische Messung an den Testelementen (24) begrenzt und dass das Testband (20) unter Zugspannung flächig auf der Führungsbahn (68) abgestützt ist, so dass das Spreitgewebe (30) spaltfrei auf der Reagenzschicht (36) anliegt, wobei eine die Führungsbahn (68) bildende Gehäusewand (76) der Applikationsspitze (18) rückseitig zu dem Messfenster (70) hin abgeschrägt ist.

2. Testbandkassette nach Anspruch 1, **dadurch gekennzeichnet, dass** die kreisbogenförmige Führungsbahn (68) einen festen Krümmungsradius vorzugsweise im Bereich von 3 bis 5 mm aufweist.

3. Testbandkassette nach Anspruch 1 oder 2, **dadurch gekennzeichnet, dass** die Führungsbahn (68) eine Längserstreckung in Bandtransportrichtung im Bereich von 5 bis 8 mm aufweist.

4. Testbandkassette nach einem der Ansprüche 1 bis 3, **dadurch gekennzeichnet, dass** die Längserstreckung der Führungsbahn (68) gleich oder kleiner als die vorzugsweise im Bereich von 5 bis 15 mm liegende Länge der Testelemente (24) ist.

5. Testbandkassette nach einem der Ansprüche 1 bis 4, **dadurch gekennzeichnet, dass** die Reagenzschicht (36) in Bandquerrichtung gesehen schmaler als das Testband (20) und breiter als das Messfenster (70) ist.

6. Testbandkassette nach einem der Ansprüche 1 bis 5, **dadurch gekennzeichnet, dass** die Applikationsspitze (18) vorzugsweise als Spritzgusspartie einstückig an dem Kassettengehäuse (50) angeformt ist und im Gebrauchszustand zum punktuellen Applizieren von Körperflüssigkeit aus dem Handgerät (12) herausragt.

## Claims

1. Test tape cassette for insertion into a hand-held device (12), in particular for blood sugar tests, comprising a cassette housing (50) for storing tape spools (54, 58), an analytical test tape (20) that can be wound forwards by means of the tape spools (54, 58), a plurality of analytical test elements (24) stored on the test tape (20) which have a spreading fabric (30) for applying body fluid and an underlying reagent layer (36) for detecting an analyte in the body fluid, wherein the cassette housing (50) has an application tip (18) that deflects the test tape (20) in order to provide the test elements (24),
**characterized in that** the application tip (18) has a guide path (68) which extends in an arch shape longitudinally to the test tape (20) and is uncurved at right angles thereto for supporting the test tape in a kink-free manner, that the apex area of the guide path (68) delimits a central opening as a measuring window (70) for an optical measurement on the test elements (24) and that the test tape (20) under tension is supported in a planar fashion on the guide path (68) such that the spreading fabric (30) lies gap-free on the reagent layer (36), wherein a housing wall (76) of the application tip (18) forming the guide path (68) is bevelled on the rear side towards the measuring window (70).

2. Test tape cassette according to claim 1, **characterized in that** the arc-shaped guide path (68) has a fixed radius of curvature preferably in the range of 3 to 5 mm.

3. Test tape cassette according to claim 1 or 2, **characterized in that** that the guide path (68) has a longitudinal extension in the direction of tape transport in the range of 5 to 8 mm.

4. Test tape cassette according to one of the claims 1 to 3, **characterized in that** the longitudinal extension of the guide path (68) is the same or less than the length of the test elements (24) which is preferably in the range of 5 to 15 mm.

5. Test tape cassette according to one of the claims 1 to 4, **characterized in that** the reagent layer (36) viewed in the tape transverse direction is narrower than the test tape (20) and wider than the measuring window (70).

6. Test tape cassette according to one of the claims 1 to 5, **characterized in** the application tip (18) is moulded in one piece preferably as an injection moulded part on the cassette housing (50) and projects from the hand-held device (12) in the operating state in order to punctually apply body fluid.

## Revendications

1. Cassette à bande de test destinée à être insérée dans un appareil à main (12), en particulier pour le test du taux de sucre dans le sang, avec un boîter de cassette (50) pour le stockage de bobines de bande (54, 58), une bande de test analytique (20) pouvant être avancée au moyen des bobines de bande (54, 58), une pluralité d'éléments de test analytiques (24) mis en réserve sur la bande de test (20), lesquels comprennent un tissu extensible (30) pour l'application de fluide corporel et une couche de réactif sous-jacente (36) pour la détection d'un analyte dans le fluide corporel, dans laquelle le boîtier de cassette (50) possède un embout d'application (18) redirigeant la bande de test (20) pour la fourniture des éléments de test (24), **caractérisée en ce que** l'embout d'application (18) comprend un chemin de guidage (68) s'étendant en forme d'arc le long de la bande de test (20) et sans courbure transversalement à celle-ci pour le support sans pli de la bande de test (20), **en ce que** le chemin de guidage (68) délimite dans sa zone de crête une percée centrale sous forme de fenêtre de mesure (70) pour une mesure optique au niveau des éléments de test (24) et **en ce que** la bande de test (20) est soutenue en tension à plat par le chemin de guidage (68) de telle sorte que le tissu extensible (30) repose sans interstice sur la couche de réactif (36), dans laquelle une paroi de boîtier (76) formant le chemin de guidage (68) de l'embout d'application (18) est biseautée à l'arrière vers la fenêtre de mesure (70).

2. Cassette à bande de test selon la revendication 1, **caractérisée en ce que** le chemin de guidage (68) en arc de cercle présente un rayon de courbure fixe compris de préférence dans la gamme allant de 3 à 5 mm.

3. Cassette à bande de test selon la revendication 1 ou la revendication 2, **caractérisée en ce que** le chemin de guidage (68) présente une extension longitudinale dans la direction de transport de bande comprise dans la gamme allant de 5 à 8 mm.

4. Cassette à bande de test selon l'une des revendications 1 à 3, **caractérisée en ce que** l'extension longitudinale du chemin de guidage (68) est égale ou inférieure à la longueur, située de préférence dans la gamme allant de 5 à 15 mm, des éléments de test (24).

5. Cassette à bande de test selon l'une des revendications 1 à 4, **caractérisée en ce que** la couche de réactif (36) vue dans la direction transversale de bande est plus étroite que la bande de test (20) et plus large que la fenêtre de mesure (70).

6. Cassette à bande de test selon l'une des revendications 1 à 5, **caractérisée en ce que** l'embout d'application (18) est conformé de préférence sous forme de partie moulée par injection d'un seul tenant au niveau du boîtier de cassette (50) et, à l'état d'utilisation pour l'application ponctuelle de fluide corporel, dépasse de l'appareil à main (12).
